(19) Europäisches Patentamt
European Patent Office
Office européen des brevets

(11) **EP 4 548 966 A1**

(12) **EUROPEAN PATENT APPLICATION**
published in accordance with Art. 153(4) EPC

(43) Date of publication:
07.05.2025 Bulletin 2025/19

(21) Application number: 22949009.9

(22) Date of filing: 13.10.2022

(51) International Patent Classification (IPC):
**A61N 2/04** (2006.01)

(86) International application number:
PCT/CN2022/125020

(87) International publication number:
WO 2024/000924 (04.01.2024 Gazette 2024/01)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: 30.06.2022 CN 202210754796

(71) Applicant: Institute of Automation, Chinese Academy Sciences
Beijing 100190 (CN)

(72) Inventors:
• **QI, Zihui**
**Beijing 100190 (CN)**
• **JIANG, Tianzai**
**Beijing 100190 (CN)**

(74) Representative: **Durm Patentanwälte PartG mbB Patentanwälte**
**Moltkestrasse 45**
**76133 Karlsruhe (DE)**

(54) **ELECTROMAGNETIC STIMULATION METHOD, APPARATUS AND DEVICE, AND READABLE STORAGE MEDIUM**

(57) The present application provides an electromagnetic stimulation method, apparatus and device, and a readable storage medium. The method is applied to the field of medical technology, and can solve the problem of poor focusing when a target area is stimulated by means of electrical stimulation or magnetic stimulation in the prior art. The method comprises: receiving an operation instruction input by a user; in a case where the operation instruction is used to instruct a deep stimulation on a target area of a target object, controlling a current direction of a TES component in the electromagnetic stimulation device in the target area to be the same as a current direction of a TMS component in the target area; and in a case where the operation instruction is used to instruct a shallow stimulation on the target area, controlling the current direction of the TES component in the target area to be opposite to the current direction of the TMS component in the target area so as to perform an electromagnetic stimulation on the target area by means of a current signal generated by the TES component and TMS in the target area. The present application can improve the spatial focusing of the electromagnetic stimulation in the target area.

FIG. 8

**Description**

CROSS-REFERENCES TO RELATED APPLICATIONS

The present application claims priority to Chinese application No. 202210754796.4 filed on June 30, 2022, entitled "Electromagnetic Stimulation Method, Apparatus and Device, and Readable Storage Medium", which is hereby incorporated by reference in its entirety.

FIELD

The present application relates to the field of medical technologies, and in particular, to an electromagnetic stimulation method, an electromagnetic stimulation apparatus, an electromagnetic stimulation apparatus and a readable storage medium.

BACKGROUND

Both transcranial electrical stimulation (TES) and transcranial magnetic stimulation (TMS) are very mature non-invasive neuromodulation techniques, which may be used for transcranial neuromodulation, as well as for stimulation of peripheral nerves and muscles, and have been widely used in clinical and research, and effectiveness of both TES and TMS has been proven.

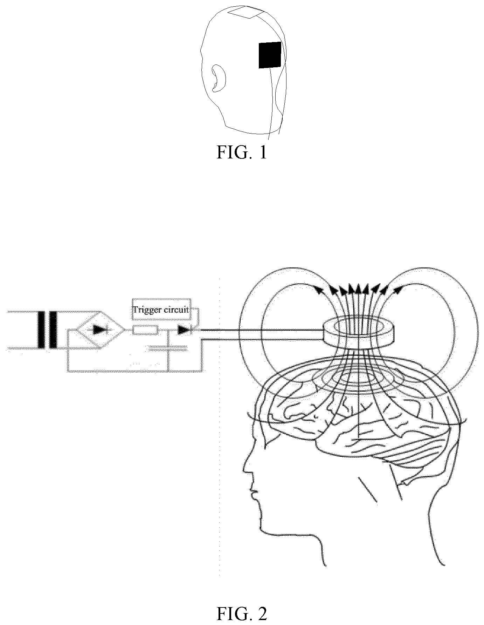

FIG. 1 is a schematic diagram of a usage way of TES in the related art. As shown in FIG. 1, a positive electrode sheet and a negative electrode sheet may be attached to a target area of a target object when TES is performed. A slight change in a cell membrane potential is caused by applying a low-intensity current in a specific pattern to the target area, thereby adjusting spontaneous discharge rates, which belongs to a subthreshold stimulation. Although TES has advantages of small size and low power consumption, TES has disadvantages of weak stimulation intensity and poor spatial focusing.

FIG. 2 is a schematic diagram of a usage way of TMS in the related art. TMS delivers strong alternating magnetic field to penetrate through a skull and induce current in the cerebral cortex to stimulate the brain nerves. Sufficiently large current may be induced in the cerebral cortex to directly activate neurons since a skin and the skull have little obstruction to the magnetic field, which belongs to a suprathreshold stimulation. TMS has advantages of high stimulation intensity, small discomfort caused, and a significant effect. TMS has disadvantages that requirement for precise neural regulation still cannot be met and it is difficult to further improve the focusing although spatial focusing of TMS is better than spatial focusing of TES.

Therefore, both TES and TMS have problems of poor spatial focusing.

BRIEF SUMMARY

The present application provides an electromagnetic stimulation method, an electromagnetic stimulation apparatus, an electromagnetic stimulation apparatus and a readable storage medium, which solve defects of insufficient spatial focusing of TMS and TES in the related art.

The present application provides an electromagnetic stimulation method, including:

receiving an operation instruction input from a user;

in response to the operation instruction, performing a control, in the case that the operation instruction is used to indicate deep stimulation of a target area of a target object, that a current direction of a pulsed transcranial electrical stimulation (TES) device of an electromagnetic stimulation apparatus inside the target area is the same as a current direction of a pulsed transcranial magnetic stimulation (TMS) device of the electromagnetic stimulation apparatus inside the target area to electromagnetically stimulate the target area through current signals generated in the target area by the TES device and the TMS device, where a stimulation intensity corresponding to the deep stimulation is greater than a first preset value; and

performing a control, in the case that the operation instruction is used to indicate shallow stimulation of the target area, that a current direction of the TES device inside the target area is opposite to a current direction of the TMS device inside the target area to electromagnetically stimulate the target area through current signals generated in the target area by the TES device and the TMS device, where a stimulation intensity corresponding to the shallow stimulation is less than or equal to the first preset value.

According to the electromagnetic stimulation method provided by the present application, the method further includes:

determining an arrangement mode of the TES device and the TMS device based on the target area and the stimulation intensity, where the stimulation intensity includes deep stimulation and shallow stimulation; and

displaying the arrangement mode of the TES device and the TMS device.

According to the electromagnetic stimulation method provided by the present application, a difference between a pulse width of a transcranial electrical stimulation (TES) and a pulse width of a transcranial magnetic stimulation (TMS) is less than a first preset value, both the pulse width of the TES and the pulse width of the TMS are less than a second preset value, a repetition frequency between a pulse of the TES and a pulse of the TMS is less than a third preset value, and the TES and the TMS are synchronized in time or differ in time by a preset value.

According to the electromagnetic stimulation method provided by the present application, phases of waveforms of the TES and the TMS are synchronized and the waveforms of the TES and the TMS are the same, and amplitude exponential decays of both the TES and the TMS are less than a fourth preset value.

According to the electromagnetic stimulation method provided by the present application, a waveform of the TES is a bipolar square wave or a bipolar triangle wave in the case that a waveform of the TMS is a bipolar waveform; and the waveform of the TES is a unipolar square wave or a unipolar triangle wave in the case that the waveform of the TMS is a unipolar waveform.

According to the electromagnetic stimulation method provided by the present application, the method further includes:

obtaining a stimulation signal for the target object during a process of electromagnetically stimulating the target area;

comparing an intensity of the stimulation signal with a second preset value to obtain a comparison result;

adjusting a magnitude of current in the TES device and/or the TMS device based on the comparison result to allow a difference between the intensity of the stimulation signal and the second preset value to be less than a preset difference.

According to the electromagnetic stimulation method provided by the present application, adjusting the magnitude of current in the TES device and the TMS device based on the comparison result includes:
in the case that the comparison result is that the difference between the intensity of the stimulation signal and the second preset value is greater than the preset difference and the intensity of the stimulation signal is less than the second preset value, increasing a magnitude of current in the pulsed TES device; and in the case that the magnitude of current in the pulsed TES device is greater than a third preset value, and the intensity of the stimulation signal is less than the second preset value, increasing a magnitude of current in the pulsed TMS device, until the difference between the intensity of the stimulation signal and the second preset value is less than the preset difference.

According to the electromagnetic stimulation method provided by the present application, adjusting the magnitude of current in the pulsed TES device and the pulsed TMS device based on the comparison result includes:
adjusting a magnitude of current in the pulsed TES device and a magnitude of current in the pulsed TMS device respectively based on a preset ratio in the case that the comparison result is that the difference between the intensity of the stimulation signal and the second preset value is greater than the preset difference.

The present application further provides an electromagnetic stimulation device, including:

a receiving module, used for receiving an operation instruction input from a user; and

a control module, used for in response to the operation instruction, performing a control, in the case that the operation instruction is used to indicate deep stimulation of a target area of a target object, that a current direction of a pulsed transcranial electrical stimulation (TES) device of an electromagnetic stimulation apparatus inside the target area is the same as a current direction of a pulsed transcranial magnetic stimulation (TMS) device of the electromagnetic stimulation apparatus inside the target area to electromagnetically stimulate the target area through current signals generated in the target area by the pulsed TES device and the pulsed TMS device, where a stimulation intensity corresponding to the deep stimulation is greater than a first preset value;

where the control module is further used for performing a control, in the case that the operation instruction is used to indicate shallow stimulation of the target area, that a current direction of the pulsed TES device inside the target area is opposite to a current direction of the pulsed TMS device inside the target area to electromagnetically stimulate the target area through current signals generated in the target area by the pulsed TES device and the pulsed TMS device, where a stimulation intensity corresponding to the shallow stimulation is less than or equal to the first preset value.

The present application further provides an electromagnetic stimulation apparatus, including: a pulsed transcranial electrical stimulation (TES) device, a pulsed transcranial magnetic stimulation (TMS) device and a controller, where the controller is connected to the pulsed TES device and the pulsed TMS device respectively, and the controller is used for performing the electromagnetic stimulation method described in any of the above embodiments.

According to the electromagnetic stimulation apparatus provided by the present application, the pulsed TES device includes a TES controller, a digital-to-analog conversion module, a current source, and multiple electrode pairs, and each electrode pair includes a positive electrode and a negative electrode;

the digital-to-analog conversion module is connected to the TES controller and a first end of the current source respectively; the digital-to-analog conversion module is used for performing digital-to-analog conversion on a control signal transmitted from the TES controller, and transmitting a converted analog signal to the current source; and

a second end of the current source is connected to a positive electrode in each electrode pair, and a third end of the current source is connected to a negative electrode in each electrode pair, and the current source is used for outputting a current to the electrode pair based on the analog signals.

According to the electromagnetic stimulation apparatus provided by the present application, the second end of the current source is connected to the positive electrode in each electrode pair through a first resistor, and the third end of the current source is connected to the negative electrode in each electrode pair through a second resistor, where a first resistor connected to a positive electrode in the same electrode pair has a same resistance value as a second resistor connected to a negative electrode in the same electrode pair, while all of resistance values of a first resistor or a second resistor corresponding to different electrode pairs are not the same.

According to the electromagnetic stimulation apparatus provided by the present application, the pulsed TES device includes a TES controller, multiple power isolation modules, multiple digital-to-analog conversion modules, multiple current sources and multiple electrode pairs, each electrode pair includes a positive electrode and a negative electrode, and the power isolation modules, the digital-to-analog conversion modules, the current sources are in one-to-one correspondence with the electrode pairs;

the TES controller is connected to a first end of each power isolation module respectively, a second end of each power isolation module is connected to a first end of a corresponding digital-to-analog conversion module, and a second end of the digital-to-analog conversion module is connected to a first end of a corresponding current source; a second end of each current source is connected to a positive electrode in a corresponding electrode pair, and a third end of the current source is connected to a negative electrode in the corresponding electrode pair;

the digital-to-analog conversion module is used for performing digital-to-analog conversion on a control signal transmitted from the TES controller, and transmitting a converted analog signal to a corresponding current source; and

the current source is used for outputting a current to a corresponding electrode pair based on the analog signals.

According to the electromagnetic stimulation apparatus provided by the present application, the pulsed TES device includes a TES controller, multiple digital-to-analog conversion modules, multiple current sources, a ground electrode and multiple electrodes, and the digital-to-analog conversion modules and the current sources are in one-to-one correspondence with the electrodes;

the TES controller is connected to a first end of each digital-to-analog conversion module, a second end of each digital-to-analog conversion module is connected to a first end of a corresponding current source, a second end of each current source is connected to a corresponding electrode, and third ends of all current sources are connected to the ground electrode;

the digital-to-analog conversion module is used for performing digital-to-analog conversion on a control signal transmitted from the TES controller, and transmitting a converted analog signal to a corresponding current source; and

the current source is used for outputting a current to a corresponding electrode based on the analog signals.

The present application further provides a non-transitory computer-readable storage medium storing a computer program, where the computer program, when executed by a processor, performs any one of the electromagnetic stimulation methods mentioned above.

In the electromagnetic stimulation method, the electromagnetic stimulation apparatus, the electromagnetic stimulation apparatus and the readable storage medium provided by the present application, the method includes: receiving an operation instruction input from a user; in response to the operation instruction, performing a control, in the case that the operation instruction is used to indicate deep stimulation of a target area of a target object, that a current direction of a pulsed TES device of an electromagnetic stimulation apparatus inside the target area is the same as a current direction of a pulsed TMS device of the electromagnetic stimulation apparatus inside the target area; or performing a control, in the case that the operation instruction is used to indicate shallow stimulation of the target area, that a current direction of the pulsed TES device inside the target area is opposite to a current direction of the pulsed TMS device inside the target area to electromagnetically stimulate the target area through current signals generated in the target area by the pulsed TES device and the pulsed TMS device. Since the current generated by the pulsed TES device and the current induced by the pulsed TMS device are superimposed on the target area simultaneously, the direction of the current of the pulsed TES device and the pulsed TMS device in the target area may be adjusted based on different needs such as deep stimulation or shallow stimulation, and the electromagnetic stimulation intensity in the target area may be increased or weakened to improve the spatial focusing of the electromagnetic stimulation in the target area and meet requirements for precise neural regulation.

BRIEF DESCRIPTION OF THE DRAWINGS

To illustrate solutions in the present application or the prior art more clearly, the drawings needed to be used in the descriptions of the embodiments or the prior art will be briefly introduced below. The drawings in the following description are some embodiments of the present application, and other drawings may be obtained based on the drawings by those skilled in the art without any creative work.

FIG. 1 is a schematic diagram of a usage way of transcranial electrical stimulation (TES) in the prior art;

FIG. 2 is a schematic diagram of a usage way of transcranial magnetic stimulation (TMS) in the prior art;

FIG. 3 is a current density curve of transcranial direct current stimulation in the prior art;

FIG. 4 is a current density curve of transcranial alternating current stimulation;

FIG. 5a is a schematic diagram of a position of an O-shaped coil above a target object;

FIG. 5b is a schematic diagram of an eddy current induced by an O-shaped coil on a target object;

FIG. 6a is a schematic diagram of a position of a figure-of-eight coil above a target object;

FIG. 6b is a schematic diagram of an eddy current induced by a figure-of-eight coil on a target object;

FIG. 7a is a schematic diagram of a position of a cone coil above a target object;

FIG. 7b is a schematic diagram of an eddy current induced by a cone coil on a target object;

FIG. 8 is a schematic flow chart of an electromagnetic stimulation method according to the present application;

FIG. 9 is a schematic diagram of eddy currents caused by electromagnetic induction;

FIG. 10 is a distribution diagram of TES electric field;

FIG. 11 is a distribution diagram of TES electric field in a human brain;

FIG. 12 is a schematic diagram of electromagnetic superposition principle in a two-dimensional plane according to the present application;

FIG. 13 is a distribution diagram of TES electric field;

FIG. 14 is a distribution diagram of TMS induced electric field;

FIG. 15 is a distribution diagram of an electromagnetic superposition electric field;

FIG. 16 is a diagram of arrangement of a single side of a circular coil and a pair of electrodes according to the present application;

FIG. 17 is a diagram of arrangement of a center line of a circular coil and a pair of electrodes according to the present application;

FIG. 18 is a diagram of arrangement of both sides of a circular coil and two pairs of electrodes according to the present application;

FIG. 19 is a diagram of arrangement of a center line of a figure-of-eight coil or a cone coil and a pair of electrodes according to the present application;

FIG. 20 is a diagram of electric field distribution in the electrode arrangement mode of FIG. 19;

FIG. 21 is a diagram of arrangement of a center line of a figure-of-eight coil or a cone coil and two pairs of electrodes according to the present application;

FIG. 22 is a diagram of arrangement of a figure-of-eight coil or a cone coil and three pairs of electrodes according to the present application;

FIG. 23 is a structural block diagram of connecting a controller to a pulsed TES device and a pulsed TMS device;

FIG. 24 is a diagram of pulse waveform of a pulsed TMS device;

FIG. 25a is a first schematic diagram of pulsed current waveforms of a pulsed TES device and a pulsed TMS device;

FIG. 25b is a second schematic diagram of pulsed current waveforms of a pulsed TES device and a pulsed TMS device;

FIG. 25c is a third schematic diagram of pulsed current waveforms of a pulsed TES device and a pulsed TMS device;

FIG. 26 is a first schematic structural diagram of a pulsed TES device according to the present application;

FIG. 27 is a second schematic structural diagram of a pulsed TES device according to the present application;

FIG. 28 is a third schematic structural diagram of a pulsed TES device according to the present application; and

FIG. 29 is a schematic structural diagram of an electromagnetic stimulation apparatus according to the present application.

## DETAILED DESCRIPTION

To illustrate objectives, solutions and advantages of the present application, the solutions of the present application are described clearly and completely below in combination with the drawings in the present application. The described embodiments are part of the embodiments of the present application, not all of them. All other embodiments obtained by those skilled in the art based on the embodiments of the present application without any creative work fall within the protection scope of the present application.

To better understand the solution of the present application, working principles of transcranial electrical stimulation (TES) and transcranial magnetic stimulation (TMS) are explained first before the solution of the present application is introduced.

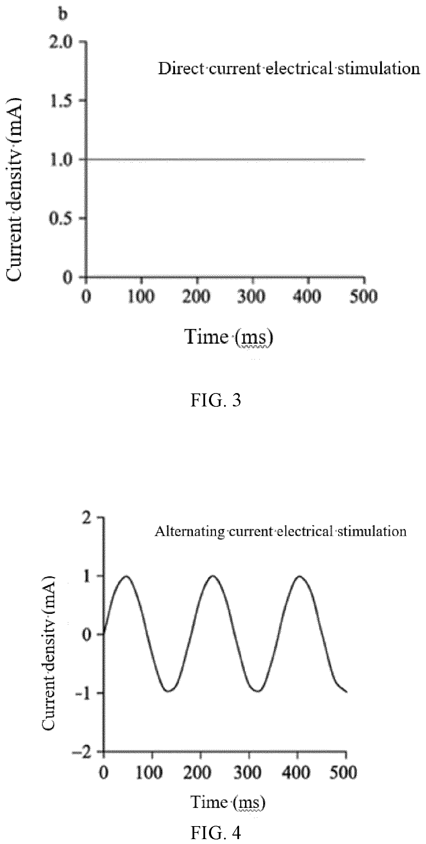

TES may apply a low-intensity current in a specific pattern to the target area, causing a slight change in a cell membrane potential, and further adjusting spontaneous discharge rates, which belongs to a subthreshold stimulation. For TES, skulls of large animals are thick, and the conductivity of the skulls is poor, resulting in more current flowing through the scalp. Simultaneously, cerebrospinal fluid has good conductivity and surrounds brain tissues, which has a strong electrical shielding effect, resulting in that the current passing through the skull is diverted by the cerebrospinal fluid, and current actually entering the cerebral cortex accounts for only a small part. Therefore, TES is low in stimulation intensity. Although high-resolution transcranial electrical stimulation and time-frequency interference electrical stimulation had been developed later, the shortcomings of weak stimulation intensity and poor focusing have not been substantially improved. High-voltage transcranial electrical stimulation may activate the nervous system non-invasively, but it will induce pain and discomfort in the scalp. Therefore, microcurrent transcranial electrical stimulation, which is usually referred to as TES, was later provided. TES may be divided into transcranial direct current stimulation (tDCS), transcranial alternating current stimulation (tACS), and transcranial random noise stimulation (tRNS), with a stimulation current of about 100 uA-5 mA. FIG. 3 is a current density curve of transcranial direct current stimulation in the prior art. As shown in FIG. 3, a current density of the transcranial direct current stimulation is usually 1.0 mA. FIG. 4 is a current density curve of transcranial alternating current stimulation. As shown in FIG. 4, an amplitude of a current density of transcranial alternating current stimulation is about 1.0 mA. In addition, TES further includes high definition-transcranial alternating current stimulation (HD-tACS), time-frequency interference electrical stimulation, etc. However, all of the above-mentioned various TES have the disadvantages of weak stimulation intensity and poor focusing.

TMS uses a principle of electromagnetic induction to generate an alternating magnetic field through the alternating current in the coil. The alternating magnetic field then generates an induced electric field in the human body, that is, eddy current (the eddy current is basically parallel to the coil plane), which stimulates the brain nerves. TMS is a painless, non-invasive and green treatment method. Sufficiently large current may be induced in the cerebral cortex to directly activate neurons since a skin and the skull have little obstruction to the magnetic field, which belongs to a suprathreshold stimulation. The transcranial magnetic stimulation has been applied in various aspects such as neuropsychology (depression, schizophrenia), rehabilitation, and pediatrics (cerebral palsy, autism, etc.) due to high stimulation intensity, small discomfort caused, and the significant effect.

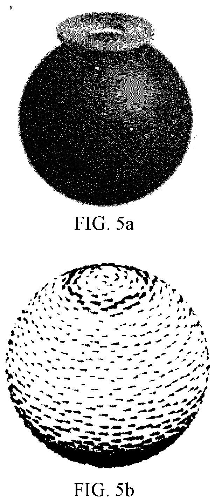

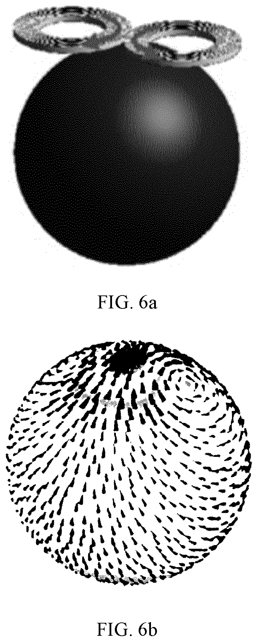

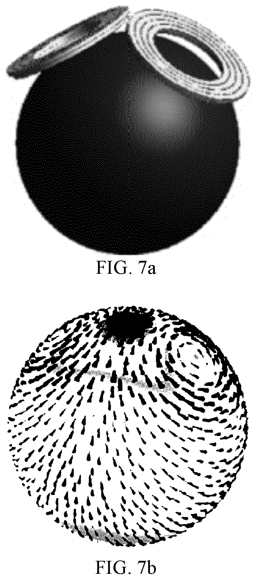

Although the spatial focusing of TMS is better than that of TES, TMS still cannot meet requirements for precise neural regulation and it is difficult to further improve the focusing. Under normal circumstances, the spatial resolution of TMS is 0.5 cm to 1 cm, and the stimulation depth is limited to 2 cm to 3 cm under the cerebral cortex. Improving the spatial focusing and penetration depth of TMS has always been the goal of users and there has been no breakthrough progress from a circular coil, to a figure-of-eight coil, further to a cone coil. FIG. 5a is a schematic diagram of a position of a O-shaped coil above a target object; FIG. 5b is a schematic diagram of an eddy current induced by a O-shaped coil on a target object; FIG. 6a is a schematic diagram of a position of a figure-of-eight coil above a target object; FIG. 6b is a schematic diagram of an eddy current induced by a figure-of-eight coil on a target object; FIG. 7a is a schematic diagram of a position of a cone coil above a target object and FIG. 7b is a schematic diagram of an eddy current induced by a cone coil on a target object. It may be seen from FIG. 5a to FIG. 7b that the figure-of-eight coil is more focused. The figure-of-eight coil uses a pair of circular coils with opposite currents to induce two groups of eddy currents in the brain. The current directions of the two groups of eddy currents are the same at the tangent point, and the current density in the target area is 2-3 times higher than that in other areas. A focal range of the figure-of-eight coil is an ellipse. The focusing performance in a line connecting the centers of the two coils is better, while the focusing performance in a tangent direction of the two coils is poor. There is an urgent need to improve the focusing in the tangent direction.

At the same time, TMS consumes huge power and it is difficult to achieve portability and wearability. In the case that the single pulse period of TMS is about 350 us, the current peak of TMS may reach 5000 A, and a short-term power of 10 Hz TMS may reach several Kw. Most of the energy is wasted in the form of heat, which also increases the burden of coil heat dissipation. Therefore, it is also a goal pursued by the industry to reduce the power consumption of TMS.

In view of the above problems, in embodiments of the present application, TES and TMS may be superimposed in space and time, and a target area of a target object may be electromagnetically stimulated by current signals generated in the superimposed area, thereby improving the spatial focusing of the electromagnetic stimulation. In addition, directions of currents in TES and TMS in the superimposed area may be changed to strengthen or weaken the intensity of electromagnetic stimulation to meet the requirements of different stimulation intensity of users.

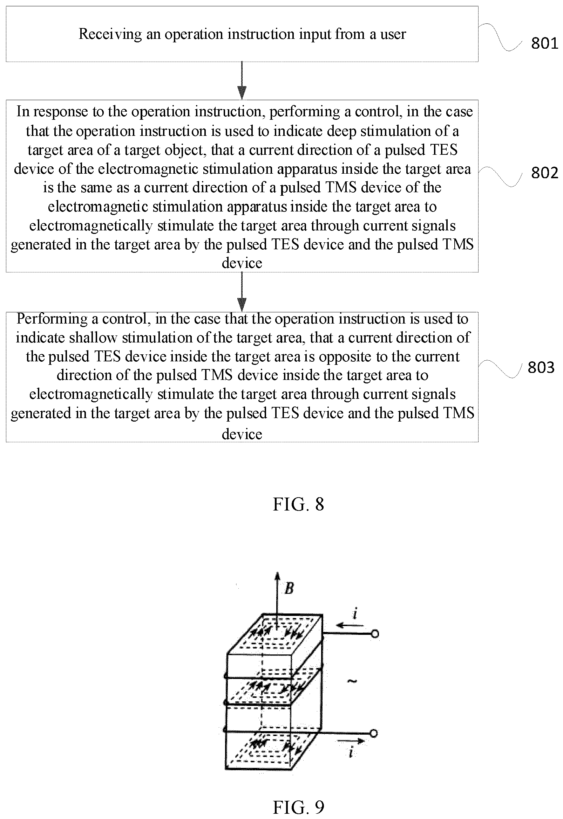

An electromagnetic stimulation method of the present application is described below in conjunction with FIG. 8. FIG. 8 is a schematic flow chart of an electromagnetic stimulation method according to the present application. As shown in FIG. 8, the method includes:

step 801: receiving an operation instruction input by a user.

Specifically, the electromagnetic stimulation method of the present application is performed with an electromagnetic stimulation apparatus, which may also be understood as a controller in the electromagnetic stimulation apparatus. The controller may control a pulsed TES device and a pulsed TMS device in the electromagnetic stimulation apparatus.

The operation instruction may be input from the user by clicking on different controls in the electromagnetic stimulation apparatus, or may be input by voice or text. For example, "deep stimulation of the transcranial region" and so on may be input.

Step 802: in response to the operation instruction, performing a control, in the case that the operation instruction is used to indicate deep stimulation of a target area of a target object, that a current direction of a pulsed TES device of the electromagnetic stimulation apparatus inside the target area is the same as a current direction of a pulsed TMS device of the electromagnetic stimulation apparatus inside the target area to electromagnetically stimulate the target area through current signals generated in the target area by the pulsed TES device and the pulsed TMS device.

The stimulation intensity corresponding to the deep stimulation is greater than a first preset value. In addition, the deep stimulation may also be understood as enhanced stimulation.

Specifically, when it is necessary to deeply stimulate the target area of the target object, it is necessary to ensure that the intensity of the electromagnetic stimulation in the target area is greater than the first preset value. In order to achieve deep stimulation, it is necessary to strengthen the electromagnetic stimulation of the target area. Due to limited penetration capability of TES, most of the current flows through a superficial cortex, and the deep current is small. By superimposing TMS in the target area, the current in the pulsed TES device and the current in the pulsed TMS device are in the same direction in the target area, and the intensity of electromagnetic stimulation in the target area is strengthened to achieving deep stimulation.

Specifically, the electromagnetic stimulation apparatus generates current in the target area of the target object through the pulsed TES device, and generates induced current in the target area of the target object through the pulsed TMS device at the same time. The current generated by the pulsed TES device and the induced current generated by the pulsed TMS device are superimposed in the target area, and the target area is electromagnetically stimulated based on the superimposed current. A principle of electromagnetic superposition is introduced as follows.

FIG. 9 is a schematic diagram of eddy currents caused by electromagnetic induction. As shown in FIG. 9, TMS uses the principle of electromagnetic induction. An alternating current in the coil generates an alternating magnetic field, and the alternating magnetic field then generates an induced electric field in the human body, that is, eddy current, which is a passive rotational field and a loop flow without a start point and an end point, and the eddy current is basically parallel to the coil plane. The figure-of-eight coil commonly used in TMS has good spatial focusing performance because the two groups of eddy currents induced by the two circular coils are added in the same direction below the middle of the two coils, and both better stimulation intensity and spatial focusing are obtained.

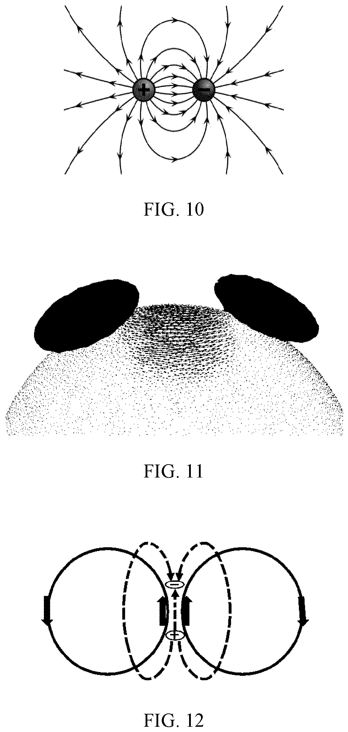

FIG. 10 is a distribution diagram of TES electric field. As shown in FIG. 10, TES transmits the electric field through the electrode, which is an active irrotational field. The current flows in one direction, always flows from a positive electrode to a negative electrode, with a start point and an end point. FIG. 11 is a distribution diagram of TES electric field in a human brain. As shown in FIG. 11, an electric field under a TES electrode is mainly perpendicular to a cortical surface, while the electric field between the electrodes is mainly tangent to a cortex.

It should be noted that the above-mentioned electrodes may be invasive electrodes or non-invasive electrodes.

It should be understood that an induced electric field of TMS and an electric field generated by current sources of TES are two independent sources, which conform to a superposition theorem. In the present application, the rotational field induced by TMS and the irrotational field of TES are superimposed in time and space, and the superimposed electric field has higher intensity and spatial focusing. The principle of electromagnetic superposition in a two-dimensional plane is first demonstrated below, and the principle of electromagnetic superposition in a three-dimensional space is then introduced.

In the two-dimensional plane, TMS and TES are used to stimulate a conductive plane, the figure-of-eight coil of TMS is tightly close to the conductive plane, and the electrodes are attached to the conductive plane, located below the figure-of-eight coil, and arranged along the tangent direction of two circles of the figure-of-eight coil. FIG. 12 is a schematic diagram of electromagnetic superposition principle in a two-dimensional plane according to the present application. As shown in FIG. 12, solid circles represent induced electric fields of the figure-of-eight coil of TMS in the conductive plane, dotted circles represent electrical stimulation electric fields of TES, and "+" and "-" represent positive and negative electrodes. In a target area formed by the "+" to "-" of TES, a current direction in TES is from a positive electrode to a negative electrode. When TMS is superimposed on the target area at the same time, a current direction induced by TMS is the same as a current direction of TES, and the current in the target area is increased. The electromagnetic stimulation of the target area may be strengthened when the current superimposed on the target area is used to stimulate the target area of the target object. The electric field strength in the non-target area is weakened since directions of the electric fields of TMS and TES are opposite outside the positive and negative electrodes.

In a three-dimensional space, when TMS and TES stimulate the brain, an electric field direction of TMS is mainly tangent to the cerebral cortex, and an electric field direction between TES electrodes is also mainly tangent to the cerebral cortex. Therefore, it also has the superposition conditions of strengthening in the same direction and weakening in the opposite direction, and the stimulation intensity and spatial focusing of the pulsed TES device and the pulsed TMS device in the target area are improved.

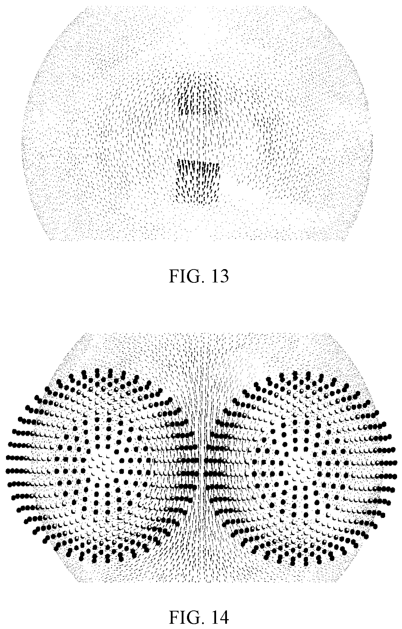

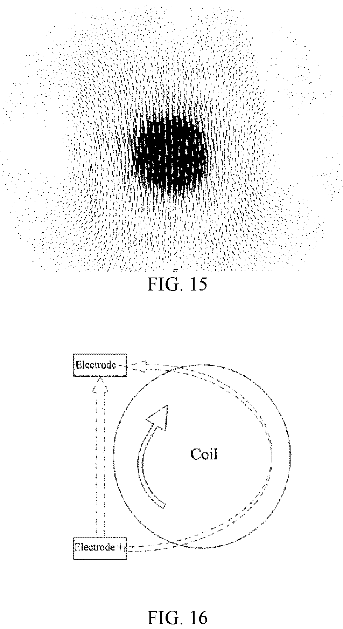

Simnibs software and ball model are used for simulation, a size of the electrode is 2 cm*2 cm, the current is 5 mA, the coil is Magstim 70 mm figure-of-eight coil, and the di/dt is 1.00x1e6 A/s. FIG. 13 is a distribution diagram of TES electric field; FIG. 14 is a distribution diagram of TMS induced electric field; and FIG. 15 is a distribution diagram of an electromagnetic superposition electric field. As shown in FIG. 13 to FIG. 15, a maximum electric field strength on a surface of a grey matter is 1.23 v/m when TES is used alone, and the maximum electric field strength on the surface of the grey matter is 1.28 v/m when TMS is used alone. After TMS and TES are superimposed, the maximum electric field strength on the surface of the grey matter reaches 2.5 v/m, which may greatly increase the stimulation intensity and focusing performance (the field strength away from the target area is greatly attenuated), especially a focal spot length along the tangent direction of the two TMS coils is smaller.

In the electromagnetic stimulation method provided in the present application, the stimulation is enhanced when an electric field direction in the target area formed between the positive and negative electrodes of TES is the same as the electric field direction induced by TMS. The stimulation is weakened since the electric field direction outside the positive and negative electrodes of TES is opposite to the electric field direction induced by TMS. The electric field strength of the target area is enhanced and the electric field strength of the non-target area is weakened to improve focusing performance. In addition, the nerves are precisely regulated by applying the superimposed current generated in the target area to the target area of the target object for electromagnetic stimulation.

Step 803: performing a control, in the case that the operation instruction is used to indicate shallow stimulation of the target area, that a current direction of a pulsed TES device inside the target area is opposite to a current direction of the pulsed TMS device inside the target area to electromagnetically stimulate the target area through current signals generated in the target area by the pulsed TES device and the pulsed TMS device.

The stimulation intensity corresponding to the shallow stimulation is less than or equal to the first preset value. In addition, the shallow stimulation may also be understood as weakened stimulation.

Specifically, when it is necessary to shallowly stimulate the target area of the target object, it is necessary to ensure that the intensity of the electromagnetic stimulation in the target area is less than or equal to the first preset value. In order to achieve shallow stimulation, it is necessary to weaken the electromagnetic stimulation of the target area. TES and TMS are superimposed in the target area so that the current direction in the pulsed TES device is opposite to the current direction in the pulsed TMS device in the target area to weaken the intensity of the electromagnetic stimulation in the target area and perform the shallow stimulation.

When the current direction in the pulsed TES device is opposite to the current direction in the pulsed TMS device in the target area, the principle of electromagnetic superposition is similar to that when the current direction in the pulsed TES device is the same as the current direction in the pulsed TMS device in the target area, and will not be repeated here.

In the case of deep stimulation, the stimulation of the target area is enhanced and the stimulation area is relatively concentrated; while a stimulation area of shallow stimulation is relatively wide, and the surface stimulation may be weakened, while the deep stimulation is retained.

In the electromagnetic stimulation method provided in the present application, by controlling currents generated by the pulsed TES device and the pulsed TMS device to be superimposed in space and time in the target area, the target area is electromagnetically stimulated by the superimposed current to improve the spatial focusing of the electromagnetic stimulation in the target area. By controlling current directions generated by the pulsed TMS device and the pulsed TES device in the target area to be the same or opposite, the intensity of the electromagnetic stimulation is also strengthened or weakened while the spatial focusing of the electromagnetic stimulation in the target area is improved to achieve deep stimulation or shallow stimulation of the target area to further improve the accuracy of nerve regulation.

Moreover, in the electromagnetic stimulation method provided in the present application, in the case that the current direction generated by the pulsed TMS device is the same as the current direction generated by the pulsed TES device in the target area, the stimulation intensity of TMS may be appropriately reduced since the total stimulation intensity is strengthened. The power consumption may be decreased to 1/4 if the TMS current may be reduced to 1/2. Therefore, the present application may significantly reduce the power consumption of TMS by superimposing the currents generated by the pulsed TMS device and the pulsed TES device in the target area in the same direction, the TMS device may be portable and wearable and application scenarios of TMS are greatly widen.

Further, on the basis of the above embodiments, in the electromagnetic stimulation apparatus, an arrangement mode of the pulsed TES device and the pulsed TMS device may also be determined based on the target area and the stimulation intensity, where the stimulation intensity includes deep stimulation and shallow stimulation; and be displayed.

Specifically, the operation instruction received by the electromagnetic stimulation apparatus includes a target area to be electromagnetically stimulated of the target object and the stimulation intensity to be achieved in the target area, where the stimulation intensity includes deep stimulation and shallow stimulation. In the process of performing deep stimulation or shallow stimulation on the target area, the arrangement mode of the pulsed TES device and the pulsed TMS device is displayed on the electromagnetic stimulation apparatus through the received operation instruction. In this way, it is convenient for users to place the pulsed TES device and the pulsed TMS device in the target area of the target object based on the displayed arrangement mode to achieve deep stimulation or shallow stimulation, thereby improving the user experience.

The arrangement mode of the pulsed TES device and the pulsed TMS device is described below specifically.

FIG. 16 is a diagram of arrangement of a single side of a circular coil and a pair of electrodes according to the present application. As shown in FIG. 16, a stimulation point of a traditional circular coil is an annular part at a lower part of the coil. If the TES electrode is located on one side of the annular ring, the stimulation intensity at one side of the annular ring may be strengthened and the stimulation intensity at another side of the annular ring may be weakened. The two sides are not equal, with one side strengthening or weakening more than another side.

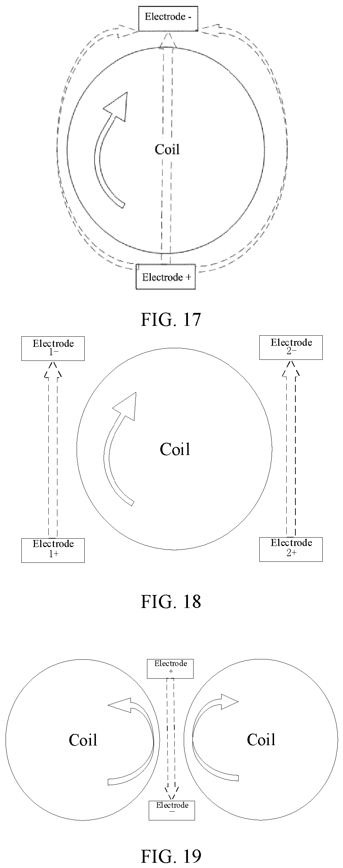

FIG. 17 is a diagram of arrangement of a center line of a circular coil and a pair of electrodes according to the present application. As shown in FIG. 17, if the TES electrode is located at the center line of an annular ring, the stimulation intensity at one side of the annular ring may be strengthened and the stimulation intensity at another side of the annular ring may be weakened. The superposition and weakening are equal.

FIG. 18 is a diagram of arrangement of both sides of a circular coil and two pairs of electrodes according to the present application. As shown in FIG. 18, two pairs of electrodes of TES are located on both sides of the circular ring respectively. By adjusting the polarity of the positive and negative electrodes, one side is strengthened and another side is weakened, or both sides are strengthened or weakened.

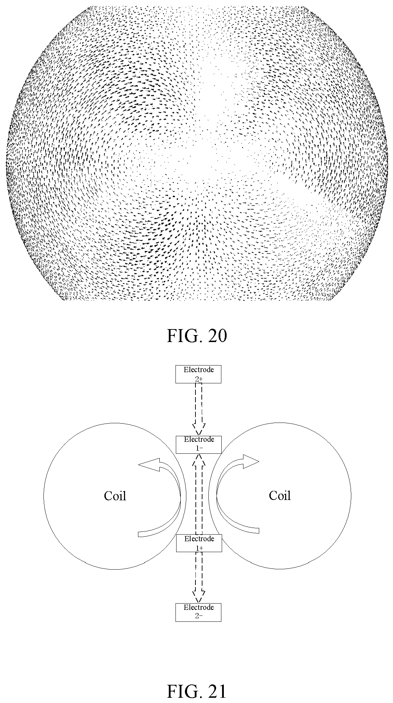

FIG. 19 is a diagram of arrangement of a center line of a figure-of-eight coil or a cone coil and a pair of electrodes according to the present application and FIG. 20 is a diagram of electric field distribution in the electrode arrangement mode of FIG. 19. As shown in FIG. 19 and FIG. 20, a pair of electrodes in a midline weakens the surface stimulation and achieves deep focusing. The electric field direction between the positive and negative electrodes of TES is opposite to an electric field direction induced by TMS, the surface stimulation is weakened. However, deep focusing is achieved because the electric field of TES decays faster with depth and the deep induced electric field of TMS is less affected by electromagnetic superposition. In addition, a double cone coil is a special figure-of-eight coil. Two circular coils are folded at an angle to produce a deeper and wider magnetic field. Compared with other TMS coils, magnetic field generated by the double cone coil decays slowly with distance to stimulate deep brain areas, but the double cone coil will activate the superficial cortex under the coil while activating the deep cortex.

FIG. 21 is a diagram of arrangement of a center line of a figure-of-eight coil or a cone coil and two pairs of electrodes according to the present application. As shown in FIG. 21, the two pairs of electrodes in the midline enhance capability to weaken an electric field strength outside the target area. An electrode with opposite polarity is added outside the midline electrode. The electric field between electrode 1+ and electrode 1- of TES is the same as the electric field induced by TMS, and the intensity is enhanced. The electric field between electrode 1+ and electrode 2-, and between electrode 1- and electrode 2+ of TES is in the opposite direction to the electric field induced by TMS, and the intensity is weakened.

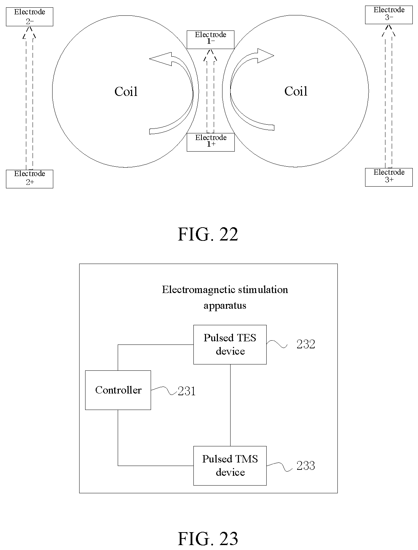

FIG. 22 is a diagram of arrangement of a figure-of-eight coil or a cone coil and three pairs of electrodes according to the present application. As shown in FIG. 22, the stimulation of the target area may be strengthened and the stimulation of the secondary side may be weakened through the three pairs of electrodes.

It should be noted that in addition to the arrangement mode of the electrodes described above, the present application also has a variety of arrangement modes of electrodes of TES to achieve different superposition effects, which are not listed here.

Further, a difference between a pulse width of a transcranial electrical stimulation (TES) and a pulse width of a transcranial magnetic stimulation (TMS) is less than a first preset value, both the pulse width of the TES and the pulse width of the TMS are less than a second preset value, a repetition frequency between a pulse of the TES and a pulse of the TMS is less than a third preset value.

FIG. 23 is a structural block diagram of connecting a controller to a pulsed TES device and a pulsed TMS device. As shown in FIG. 23, the pulse width of the TES and the pulse width of the TMS and a repetition frequency may be controlled by the main controller electrically connected thereto. The main controller may ensure that pulse widths output from the TES and TMS are the same or similar, by controlling the difference between the pulse width of the TES and the pulse width of the TMS to be less than the first preset value. The main controller may ensure that waveforms of the currents superimposed in the target area of the pulsed TES device and the pulsed TMS device are the same or similar, and may also ensure that the pulse widths of the pulsed TES device and the pulsed TMS device may effectively stimulate the target area, by controlling both the pulse width of the TES and the pulse width of the TMS to be less than the second preset value and the repetition frequency between a pulse of the TES and a pulse of the TMS to be less than the third preset value. Usually, pulse widths of the TES and the TMS are very short, generally less than 500 microseconds.

In the electromagnetic stimulation method provided in the present application, the difference between the pulse width of the TES and the pulse width of the TMS is less than the first preset value, both the pulse width of the TES and the pulse width of the TMS are less than the second preset value, the repetition frequency between a pulse of the TES and a pulse of the TMS is less than the third preset value, and the TES and the TMS are synchronized in time or differ in time by a preset value and thus waveforms of the currents superimposed in the target area of the pulsed TES device and the pulsed TMS device are the same or similar and the stimulation intensity and spatial focusing of the electromagnetic stimulation in the target area are improved. In addition, since the pulse width of the TES and the pulse width of the TMS are less than the second preset value (less than 500 microseconds), a current peak value of the TES that the human body may withstand may be greatly increased, from 5 mA of the traditional continuous TES to 50 mA. A volume of the pulsed TMS device is further reduced and it is more convenient to carry the TMS since the current peak value of the TES may be increased.

Further, phases of waveforms of the TES and the TMS may be synchronized and the waveforms of the TES and the TMS may be the same, and amplitude exponential decays of both the TES and the TMS are less than the fourth preset value.

Specifically, to effectively superpose currents of the pulsed TES device and the pulsed TMS device in the target area, it is necessary to ensure that the currents of the pulsed TES device and the pulsed TMS device have the same or similar waveforms. A measure for ensuring that the currents of the pulsed TES device and the pulsed TMS device have the same or similar waveforms includes: the phases of the TES and the TMS are the same, and amplitude exponential decays of both the TES and the TMS are less than the fourth preset value. The waveforms of currents generated by TMS and TES are adjusted through a pulse current source. That is, the current generated by TMS and the current generated by TES have the same or similar waveforms through the pulse current source.

As an example, the above waveforms may include sine waves and cosine waves.

Further, a waveform of the TES is a bipolar square wave or a bipolar triangle wave in the case that a waveform of the TMS is a bipolar waveform; and the waveform of the TES is a unipolar square wave or a unipolar triangle wave in the case that the waveform of the TMS is a unipolar waveform.

Specifically, a waveform of TES may be set to a bipolar square wave, a bipolar triangle wave, a unipolar square wave or a unipolar triangle wave to approximate a waveform of TMS, the situation where the waveforms of TMS and TES must be consistent is avoided, and the application range of electromagnetic stimulation is wider.

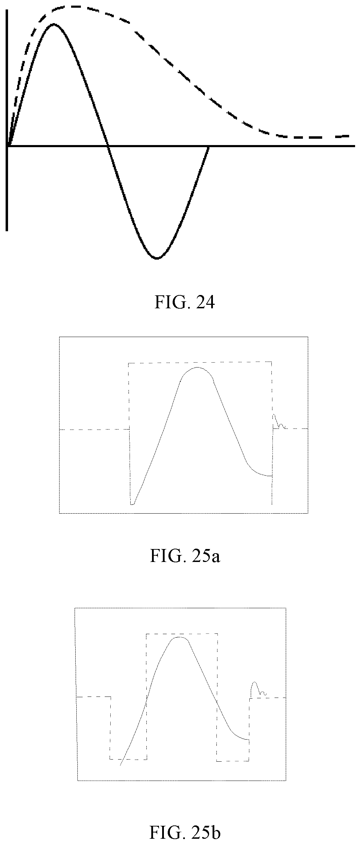

FIG. 24 is a diagram of pulse waveform of a pulsed TMS. As shown in FIG. 24, the pulsed TMS may be a bipolar pulse Biphasic (shown by a solid line) or a monopolar pulse Monophasic (shown by a dotted line). It may be seen from FIG. 24 that the waveform of the cosine pulse of TES has the same amplitude exponential decay as a TMS induced current. When TES and TMS have the same or similar waveform, amplitude, and attenuation, the electromagnetic stimulation effect of both on the target area is the best.

How to synchronize the pulsed TMS and TES is illustrated below by taking the commonly used bipolar pulse Biphasic as an example.

The pulsed TES device uses a pulse current source. The pulse current source may avoid the influence of the voltage induced by the pulsed TMS. The waveform and phase of the pulse current source may be exactly the same as or different from the TMS induced electric field.

A current function of the bipolar TMS coil is:

$$I_L(t) = \frac{V_C(0)}{\omega L} \text{Sin}(\omega t) \exp(-\alpha t), t \geq 0$$

where $V_C(0)$ is an initial capacitor voltage, $\omega$ is an angular frequency, L is an inductance, $\alpha$ is a decay rate, and t is time.

The current $I_{TMS}$ (t) induced by TMS in the human body is simplified expressed by:

$$I_{TMS}(t) = I_{TMS}(0)\text{Cos}(\omega t) \exp(-\alpha t), t \geq 0$$

where $I_{TMS}(0)$ is the peak value of the TMS induced current $I_L(t)$, which is associated with a coil distance, a coil strength, and characteristics of to be tested tissue.

The main controller may synchronize the TMS with the TES by controlling the above parameters of current generated by the pulsed TES device to be consistent with the corresponding parameters of current generated by the pulsed TMS device.

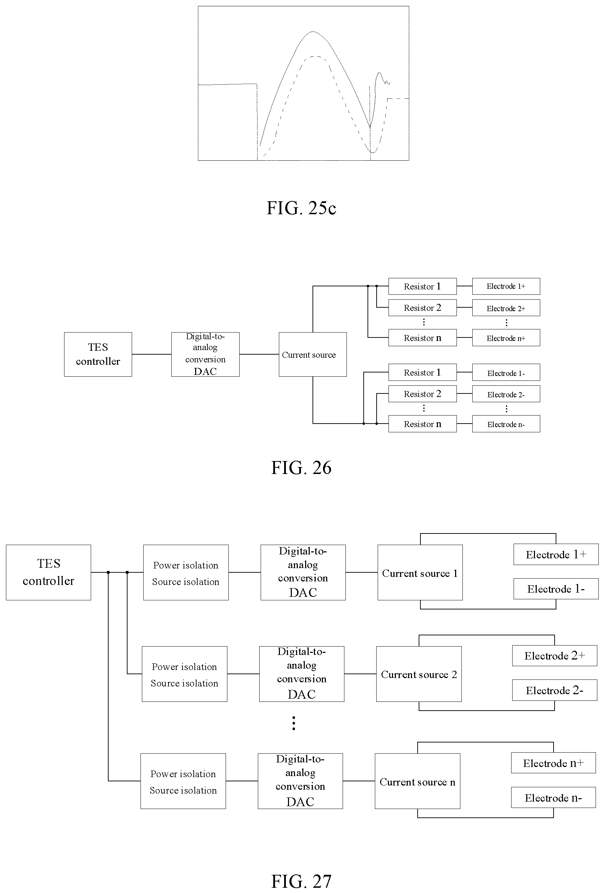

FIG. 25a is a first schematic diagram of pulsed current waveforms of a pulsed TES device and a pulsed TMS device; FIG. 25b is a second schematic diagram of pulsed current waveforms of a pulsed TES device and a pulsed TMS device; and FIG. 25c is a third schematic diagram of pulsed current waveforms of a pulsed TES device and a pulsed TMS device. The solid line is a voltage waveform induced by the bipolar TMS coil current, and is a single pulse cosine waveform with an exponentially decaying amplitude. The pulsed TES device and the pulsed TMS device are triggered synchronously to achieve strict time synchronization. Waveform data of the TES is prestored in the main controller, and the main controller may output the current waveform when receiving the trigger signal.

In the electromagnetic stimulation method provided in the present application, phases of waveforms of the TES and the TMS may be the same, and amplitude exponential decays of both the TES and the TMS are less than the fourth preset value, and thus currents generated by the pulsed TES device and the pulsed TMS device are effectively superimposed in the target area and the stimulation intensity and spatial focusing of the electromagnetic stimulation in the target area are finally improved.

Further, in the process of electromagnetically stimulating the target area of the target object, a magnitude of current in the pulsed TES device and/or the pulsed TMS device may be adjusted based on a stimulation signal during the stimulation process, and thus the intensity of the electromagnetic stimulation is closer to the actual required intensity.

As an example, a stimulation signal of the target object during the process of electromagnetically stimulating the target area may be obtained, and an intensity of the stimulation signal may be compared with the second preset value to obtain the comparison result, and the magnitude of current in the pulsed TES device and/or the pulsed TMS device is adjusted based on the comparison result to allow a difference between the intensity of the stimulation signal and the second preset value is less than the preset difference.

Specifically, the present application measures an effect of electromagnetic stimulation on the target object through the stimulation signal. When the difference between the intensity of the stimulation signal and the second preset value is less than the preset difference, it means that the stimulation intensity of the electromagnetic stimulation on the target object is close to the preset stimulation intensity. Therefore, the electromagnetic stimulation apparatus determines whether the current intensity of electromagnetic stimulation is appropriate by obtaining the intensity of the stimulation signal and comparing the intensity of the stimulation signal with the second preset value. It means that the intensity of electromagnetic stimulation is too large or too small if the difference between the intensity of the stimulation signal and the second preset value is greater than the preset difference. At this time, it is necessary to adjust the magnitude of current in the pulsed TES device in the target area, or adjust the magnitude of current in the pulsed TMS device in the target area, or jointly adjust magnitudes of current in the pulsed TES device and the pulsed TMS device in the target area, thereby adjusting the intensity of electromagnetic stimulation.

It should be understood that the above-mentioned stimulation signal may be, for example, an electromyographic signal, an electroencephalogram signal, or a cerebral blood oxygen signal.

In the electromagnetic stimulation method provided in the present application, by adjusting the magnitude of current in the pulsed TES device and/or the pulsed TMS device to allow the difference between the intensity of the stimulation signal and the second preset value is less than the preset difference, the intensity of the adjusted electromagnetic stimulation is close to the preset stimulation intensity, thereby avoiding a phenomenon that the stimulation of the target area does not produce a substantial stimulation effect since the stimulation intensity is too small and, or a poor user experience is caused since the stimulation intensity is too large. In addition, the accuracy of electromagnetic stimulation of the target area of the target object is improved since the stimulation intensity may be adjusted in real time during the process of electromagnetically stimulate the target area.

Further, based on the above embodiments, the magnitude of current in the pulsed TES device and the pulsed TMS device is adjusted based on the comparison result in the present application. In a possible implementation, a magnitude of current in the pulsed TES device is increased in the case that the comparison result is that the difference between the intensity of the stimulation signal and the second preset value is greater than the preset difference, and the intensity of the stimulation signal is less than the second preset value; and a magnitude of current in the pulsed TMS device is increased in the case that the magnitude of current in the pulsed TES device is greater than a third preset value, and the intensity of the stimulation signal is less than the second preset value, until the difference between the intensity of the stimulation signal and the second preset value is less than the preset difference.

Specifically, it means that the intensity of the electromagnetic stimulation is not large enough to have a substantial effect on the target object when the intensity of the stimulation signal is less than the second preset value. Therefore, it is necessary to increase the stimulation until the difference between the intensity of the stimulation signal and the second preset value is less than the preset difference, and the electromagnetic stimulation may have a substantial effect on the target object. When the intensity of stimulation is increased, in order to reduce power consumption, the present application first adjusts the current in the pulsed TES device to allow the difference between the intensity of the stimulation signal and the second preset value to be less than the preset difference since magnetic stimulation consumes more power than electrical stimulation. The target object will experience discomfort in the case that the magnitude of current in the pulsed TES device is greater than the third preset value. Therefore, the magnitude of current in the pulsed TES device is preferentially adjusted in the case that the magnitude of current in the pulsed TES device is less than the third preset value. The magnitude of current in the pulsed TMS device is increased to allow the difference between the intensity of the stimulation signal and the second preset value to be less than the preset difference only when the magnitude of current in the pulsed TES device is greater than the third preset value.

In the electromagnetic stimulation method provided in the present application, the target object is effectively stimulated by an adjusting mode of preferentially increasing the magnitude of current in the pulsed TES device through comparing the difference between the stimulation signal and the second preset value and comparing the intensity of the stimulation signal with the second preset value, which may ensure that the power consumption of the electromagnetic stimulation apparatus is reduced as much as possible in the case that the target object is effectively stimulated.

In another possible implementation, a magnitude of current in the pulsed TMS device is decreased in the case that the comparison result is that the difference between the intensity of the stimulation signal and the second preset value is greater than the preset difference, and the intensity of the stimulation signal is greater than the second preset value; and a magnitude of current in the pulsed TES device is decreased in the case that the magnitude of current in the pulsed TMS device is less than a fourth preset value, and the intensity of the stimulation signal is greater than the second preset value, until the difference between the intensity of the stimulation signal and the second preset value is less than the preset difference.

Specifically, the present adjusting mode is contrary to the previous adjusting mode, and the previous adjusting mode is to effectively stimulate the target object by strengthening the stimulation. The present adjusting mode is to effectively stimulate the target object by reducing the stimulation. It means that the intensity of the electromagnetic stimulation to the target object is too large, and the intensity of the electromagnetic stimulation needs to be decreased in the case that the intensity of the stimulation signal is greater than the second preset value. It means that the electromagnetic stimulation may effectively stimulate the target object when the intensity of the electromagnetic stimulation is decreased to the comparison result that the difference between the intensity of the stimulation signal and the second preset value is less than the preset difference. In the process of reducing the stimulation intensity, in order to ensure effective stimulation of the target object, the difference between the intensity of the stimulation signal and the second preset value is less than the preset difference by preferentially decreasing the magnitude of current in the pulsed TMS device. However, it means that the pulsed TMS device cannot achieve effective stimulation of the target object in the case that the magnitude of current in the pulsed TMS device is less than the fourth preset value. Then, it is necessary to ensure effective stimulation of the target object by the pulsed TMS device by adjusting the magnitude of current in the pulsed TES device. It means that the intensity of electromagnetic stimulation may achieve effective stimulation of the target object in the case that the magnitude of current in the pulsed TES device is adjusted to allow the difference between the intensity of the stimulation signal and the second preset value to be less than the preset difference and then stop decreasing the magnitude of current in the pulsed TES device.

In the electromagnetic stimulation method provided in the present application, the target object is effectively stimulated by an adjusting mode of preferentially decreasing the magnitude of current in the pulsed TES device through the comparison result that the difference between the intensity of the stimulation signal and the second preset value is greater than the preset difference and the intensity of the stimulation signal is greater than the second preset value, which may ensure that the power consumption of the electromagnetic stimulation apparatus is reduced as much as possible in the case that the target object is effectively stimulated.

In still another implementation, a magnitude of current in the pulsed TES device and the pulsed TMS device may be adjusted respectively based on a preset ratio in the case that the comparison result is that the difference between the intensity of the stimulation signal and the second preset value is greater than the preset difference.

Specifically, the difference between the intensity of the stimulation signal and the second preset value is less than the preset difference by jointly adjusting the magnitudes of current in the pulsed TES device and the pulsed TMS device in the case that the comparison result is that the difference between the intensity of the stimulation signal and the second preset value is greater than the preset difference. The adjusting mode may include adjusting the magnitudes of current in the pulsed TES device and the pulsed TMS device respectively based on the preset ratio to effectively stimulate the target object. For example, the preset ratio may be 1:4, 3:7, 4:6, etc., and the present application is not limited thereto as long as the target object may be stimulated as quickly and effectively as possible.

In the electromagnetic stimulation method provided in the embodiment of the present application, the magnitudes of current in the pulsed TES device and the pulsed TMS device are adjusted respectively by a preset ratio at the same time, and thus the difference between the intensity of the stimulation signal and the second preset value may be less than the preset difference in the shortest time, thereby improving the efficiency of implementing effective electromagnetic stimulation on the target object.

In still another possible implementation, a prompt message is output in the case that the comparison result is that the difference between the intensity of the stimulation signal and the second preset value is greater than the preset difference and the prompt message is used to remind a user to adjust a placement position of the pulsed TES device and/or the pulsed TMS device.

Specifically, in the case that the placement position of the pulsed TES device or the pulsed TMS device in the target area has a relatively large error range, the placement position of the pulsed TES device and/or the pulsed TMS device is preferentially adjusted to effectively stimulate the target object, thereby achieving the efficiency of implementing effective electromagnetic stimulation on the target object. The placement position of the pulsed TES device and/or the pulsed TMS device may be adjusted by referring to the electrode arrangement mode of FIG. 16 to FIG. 22, which will not be repeated here.

The present application further provides an electromagnetic stimulation apparatus. FIG. 23 is a structural block diagram of a main controller, a pulsed TES device, and a pulsed TMS device. As shown in FIG. 23, the electromagnetic stimulation apparatus includes a pulsed TES device 232, a pulsed TMS device 233, and a controller 231. The controller 231 is electrically connected to the pulsed TES device 232 and the pulsed TMS device 233, respectively. The controller 231 may perform the electromagnetic stimulation method described in any of the above embodiments.

Further, based on the embodiment of FIG. 23, the present application provides structural diagrams of three types of TES devices: namely, a single-channel current source multi-electrode type, an isolated multi-channel current source type, and a non-isolated multi-channel current source type. The three current sources may all generate an electric field with the same waveform as the TMS induced voltage, and the waveforms of multi-channel current sources are the same, but the amplitudes may be different to achieve a better superposition effect.

FIG. 26 is a first schematic structural diagram of a pulsed TES device according to the present application. As shown in FIG. 26, the pulsed TES device includes a TES controller, a digital-to-analog conversion module, a current source, and multiple electrode pairs, each electrode pair including a positive electrode and a negative electrode.

The digital-to-analog conversion module is connected to the TES controller and a first end of the current source respectively; the digital-to-analog conversion module is used to perform digital-to-analog conversion on the control signals transmitted from the TES controller, and transmit a converted analog signal to the current source. A second end of the current source is connected to a positive electrode in each electrode pair, and a third end of the current source is connected to a negative electrode in each electrode pair, and the current source is used to output current to the electrode pair based on the analog signals.

Specifically, the pulsed TES device provided by the present application is a single-channel current source multi-electrode type, which uses one current source to drive multiple electrode pairs, and each electrode pair may be connected in series with resistors of different resistance values. The resistors are used to adjust a distribution ratio of the current between different electrode pairs, and the electrode pairs may be flexibly used and arranged according to the actual situation of the target object.

In the electromagnetic stimulation apparatus provided by the present application, the electrode pairs may be flexibly used and arranged according to the actual situation of the target object to improve the stimulation efficiency of the target area since the pulsed TES device of single-channel current source multi-electrode type includes multiple electrode pairs with different magnitudes of current.

Further, in the electromagnetic stimulation apparatus provided by the present application, as shown in FIG. 26, the second end of the current source is connected to the positive electrode in each electrode pair through a first resistor, and the third end of the current source is connected to the negative electrode in each electrode pair through a second resistor, where a first resistor connected to a positive electrode in the same electrode pair has a same resistance value as a second resistor connected to a negative electrode in the same electrode pair, and all of resistance values of a first resistor or a second resistor corresponding to different electrode pairs are not the same.

In the electromagnetic stimulation apparatus provided by the present application, different electrode pairs may output different currents to provide electromagnetic stimulation of different stimulation intensities, which provides users with multiple choices and increases the flexibility of use, since all of the resistance values of the first resistor or the second resistor corresponding to different electrode pairs are not the same.

FIG. 27 is a second schematic structural diagram of a pulsed TES device according to the present application. As shown in FIG. 27, the pulsed TES device includes a TES controller, multiple power isolation modules, multiple digital-to-analog conversion modules, multiple current sources and multiple electrode pairs, each electrode pair includes a positive electrode and a negative electrode, and the power isolation modules, the digital-to-analog conversion modules, the current sources are in one-to-one correspondence with the electrode pairs; the TES controller is connected to a first end of each power isolation module respectively, a second end of each power isolation module is connected to a first end of a corresponding digital-to-analog conversion module, and a second end of the digital-to-analog conversion module is connected to a first end of a corresponding current source; a second end of each current source is connected to a positive electrode in a corresponding electrode pair, and a third end of the current source is connected to a negative electrode in the corresponding electrode pair; the digital-to-analog conversion module is used for performing digital-to-analog conversion on a control signal transmitted from the TES controller, and transmitting a converted analog signal to a corresponding current source; and the current source is used for outputting a current to a corresponding electrode pair based on the analog signals.

In the electromagnetic stimulation apparatus provided by the present application, each pair of electrodes is driven by an isolated current source since the pulsed TES device is an isolated multi-channel current source type. The current of each pair of electrodes may be accurately controlled without interference from other channels, thereby improving the accuracy of electromagnetic stimulation since the channels are electrically isolated.

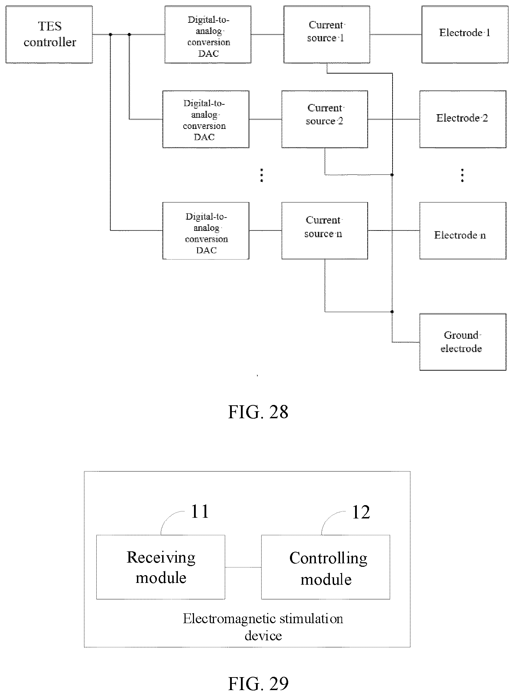

FIG. 28 is a third schematic structural diagram of a pulsed TES device according to the present application. As shown in FIG. 28, the pulsed TES device includes a TES controller, multiple digital-to-analog conversion modules, multiple current sources, multiple electrodes and a ground electrode and multiple electrodes include positive electrodes and negative electrodes, and the digital-to-analog conversion modules and the current sources are in one-to-one correspondence with the electrodes; the TES controller is connected to a first end of each digital-to-analog conversion module, a second end of each digital-to-analog conversion module is connected to a first end of a corresponding current source, a second end of each current source is connected to a corresponding electrode, and third ends of all current sources are connected to the ground electrode; the digital-to-analog conversion module is used for performing digital-to-analog conversion on a control signal transmitted from the TES controller, and transmitting a converted analog signal to a corresponding current source; and the current source is used for outputting a current to a corresponding electrode pair based on the analog signals.

In the electromagnetic stimulation apparatus provided by the present application, multiple current sources share a common ground electrode or a balanced electrode since each electrode is driven by one non-isolated current source. By accurately configuring the amplitude of each current source, the sum of all current sources is 0, thereby achieving the purpose of accurately allocating the current to each electrode. The ground electrode is used to balance additional current caused by the current error when the sum of the current sources is not 0. Taking channel 2 as an example, current source 1 is set to 5 mA and connected to electrode 1+, and current source 2 is set to -5 mA and connected to electrode 1-. At this time, no current passes through the ground electrode, and the current of electrode 1+ completely flows through electrode 1-.

FIG. 29 is a schematic structural diagram of an electromagnetic stimulation apparatus according to the present application. As shown in FIG. 29, the apparatus includes:

a receiving module 11, used for receiving an operation instruction input from a user; and

a control module 12, used for in response to the operation instruction, performing a control, in the case that the operation instruction is used to indicate deep stimulation of a target area of a target object, that a current direction of a pulsed transcranial electrical stimulation (TES) device of an electromagnetic stimulation apparatus inside the target area is the same as a current direction of a pulsed transcranial magnetic stimulation (TMS) device of the electromagnetic stimulation apparatus inside the target area to electromagnetically stimulate the target area through current signals generated in the target area by the pulsed TES device and the pulsed TMS device, where a stimulation intensity corresponding to the deep stimulation is greater than a first preset value;

where the control module 12 is further used for performing a control, in the case that the operation instruction is used to indicate shallow stimulation of the target area, that a current direction of the pulsed TES device inside the target area is opposite to a current direction of the pulsed TMS device inside the target area to electromagnetically stimulate the target area through current signals generated in the target area by the pulsed TES device and the pulsed TMS device, where a stimulation intensity corresponding to the shallow stimulation is less than or equal to the first preset value.

In an embodiment, the apparatus further includes:

a determining module, used for determining an arrangement mode of the pulsed TES device and the pulsed TMS device based on the target area and the stimulation intensity, where the stimulation intensity includes deep stimulation and shallow stimulation; and

a displaying module, used for displaying the arrangement mode of the pulsed TES device and the pulsed TMS device.

Alternatively, a difference between a pulse width of a transcranial electrical stimulation (TES) and a pulse width of a transcranial magnetic stimulation (TMS) is less than a first preset value, both the pulse width of the TES and the pulse width of the TMS are less than a second preset value, a repetition frequency between a pulse of the TES and a pulse of the TMS is less than a third preset value, and the TES and the TMS are synchronized in time or differ in time by a preset value.

Alternatively, phases of waveforms of the TES and the TMS are synchronized and the waveforms of the TES and the TMS may be the same, and amplitude exponential decays of both the TES and the TMS are less than a fourth preset value.

Alternatively, a waveform of the TES is a bipolar square wave or a bipolar triangle wave in the case that a waveform of the TMS is a bipolar waveform; and the waveform of the TES is a unipolar square wave or a unipolar triangle wave in the case that the waveform of the TMS is a unipolar waveform.

In an embodiment, the apparatus further includes:

an obtaining module, used for obtaining a stimulation signal for the target object during a process of electromagnetically stimulating the target area;

a comparing module, used for comparing an intensity of the stimulation signal with a second preset value to obtain a comparison result;

an adjusting module, used for adjusting a magnitude of current in the pulsed TES device and/or the pulsed TMS device based on the comparison result to allow a difference between the intensity of the stimulation signal and the second preset value to be less than a preset difference.

In an embodiment, the adjusting module is used for:
in the case that the comparison result is that the difference between the intensity of the stimulation signal and the second preset value is greater than the preset difference and the intensity of the stimulation signal is less than the second preset value, increasing a magnitude of current in the pulsed TES device; and in the case that the magnitude of current in the pulsed TES device is greater than a third preset value, and the intensity of the stimulation signal is less than the second preset value, increasing a magnitude of current in the pulsed TMS device, until the difference between the intensity of the stimulation signal and the second preset value is less than the preset difference.

In an embodiment, the adjusting module is used for:
adjusting a magnitude of current in the pulsed TES device and the pulsed TMS device respectively based on a preset ratio in the case that the comparison result is that the difference between the intensity of the stimulation signal and the second preset value is greater than the preset difference.

The apparatus of the present embodiment may be used to perform the method of any embodiment in the aforementioned electromagnetic stimulation methods, and its specific implementation process and effects are similar to those in the embodiments of the electromagnetic stimulation methods. For details, the detailed descriptions in the embodiments of the electromagnetic stimulation methods can be referred to, which will not be repeated here.

The present application further provides a non-transitory computer-readable storage medium storing a computer program, where the computer program, when executed by a processor, performs the electromagnetic stimulation method described in embodiments above. The method includes:
receiving an operation instruction input from a user; in response to the operation instruction, performing a control, in the case that the operation instruction is used to indicate deep stimulation of a target area of a target object, that a current direction of a pulsed transcranial electrical stimulation (TES) device of an electromagnetic stimulation apparatus inside the target area is the same as a current direction of a pulsed transcranial magnetic stimulation (TMS) device of the electromagnetic stimulation apparatus inside the target area to electromagnetically stimulate the target area through current signals generated in the target area by the pulsed TES device and the pulsed TMS device, where a stimulation intensity corresponding to the deep stimulation is greater than the first preset value; and performing a control, in the case that the operation instruction is used to indicate shallow stimulation of the target area, that a current direction of the pulsed TES device inside the target area is opposite to a current direction of the pulsed TMS device inside the target area to electromagnetically stimulate the target area through current signals generated in the target area by the pulsed TES device and the pulsed TMS device, where a stimulation intensity corresponding to the shallow stimulation is less than or equal to the first preset value.

The apparatus embodiments described above are merely illustrative, wherein the units described as separate elements may or may not be physically separated, and the elements displayed as units may or may not be physical units, that is, may be located at the same place or be distributed to multiple network units. Some or all of the modules may be selected according to actual needs to achieve the purpose of the solution of the present embodiment. Those skilled in the art may understand and implement the embodiments described above without paying creative labors.

Through the description of the embodiments above, those skilled in the art may clearly understand that the various embodiments may be implemented by means of software and a necessary general hardware platform, and of course, by hardware. Based on such understanding, the technical solution of the present application or a part of the technical solution, which is essential or contributes to the prior art, may be embodied in the form of a software product, which is stored in a storage medium such as ROM/RAM, magnetic Discs, optical discs, etc., including several instructions to cause a computer device (which may be a personal computer, server, or network device, etc.) to perform various embodiments or a part of the methods described in various embodiments.

It should be noted that the above embodiments are only used to explain the solutions of the present application, and are not limited thereto; although the present application has been described in detail with reference to the foregoing embodiments, it should be understood by those skilled in the art that modifications to the solutions documented in the foregoing embodiments and equivalent substitutions to a part of the features may be made and these modifications and substitutions do not make corresponding solutions depart from the scope of the solutions of various embodiments of the present application.

**Claims**

1. An electromagnetic stimulation method, comprising:

    receiving an operation instruction input by a user;
    in response to the operation instruction, performing a control, in the case that the operation instruction is used to indicate deep stimulation of a target area of a target object, that a current direction of a pulsed transcranial electrical stimulation (TES) device of an electromagnetic stimulation apparatus inside the target area is the same as a current direction of a pulsed transcranial magnetic stimulation (TMS) device of the electromagnetic stimulation apparatus inside the target area to electromagnetically stimulate the target area through current signals generated in the target area by the pulsed TES device and the pulsed TMS device, wherein a stimulation intensity corresponding to the deep stimulation is greater than a first preset value; and

perform a control, in the case that the operation instruction is used to indicate shallow stimulation of the target area, that a current direction of the pulsed TES device inside the target area is opposite to a current direction of the pulsed TMS device inside the target area to electromagnetically stimulate the target area through current signals generated in the target area by the pulsed TES device and the pulsed TMS device, wherein a stimulation intensity corresponding to the shallow stimulation is less than or equal to the first preset value.

2. The method of claim 1, further comprising:

determining an arrangement mode of the pulsed TES device and the pulsed TMS device based on the target area and the stimulation intensity, wherein the stimulation intensity comprises deep stimulation and shallow stimulation; and

displaying the arrangement mode of the pulsed TES device and the pulsed TMS device.

3. The method of claim 1, wherein a difference between a pulse width of a transcranial electrical stimulation (TES) and a pulse width of a transcranial magnetic stimulation (TMS) is less than a first preset value, both the pulse width of the TES and the pulse width of the TMS are less than a second preset value, a repetition frequency between a pulse of the TES and a pulse of the TMS is less than a third preset value, and the TES and the TMS are synchronized in time or differ in time by a preset value.

4. The method of claim 3, wherein phases of waveforms of the TES and the TMS are synchronized and the waveforms of the TES and the TMS are the same, and amplitude exponential decays of both the TES and the TMS are less than a fourth preset value.

5. The method of claim 3, wherein a waveform of the TES is a bipolar square wave or a bipolar triangle wave in the case that a waveform of the TMS is a bipolar waveform; and the waveform of the TES is a unipolar square wave or a unipolar triangle wave in the case that the waveform of the TMS is a unipolar waveform.

6. The method of claim 3 or 4, further comprising:

obtaining a stimulation signal for the target object during a process of electromagnetically stimulating the target area;

comparing an intensity of the stimulation signal with a second preset value to obtain a comparison result;

adjusting a magnitude of current in the pulsed TES device and/or the pulsed TMS device based on the comparison result to allow a difference between the intensity of the stimulation signal and the second preset value to be less than a preset difference.

7. The method of claim 6, wherein adjusting the magnitude of current in the pulsed TES device and the pulsed TMS device based on the comparison result comprises:

in the case that the comparison result is that the difference between the intensity of the stimulation signal and the second preset value is greater than the preset difference and the intensity of the stimulation signal is less than the second preset value, increasing a magnitude of current in the pulsed TES device; and in the case that the magnitude of current in the pulsed TES device is greater than a third preset value, and the intensity of the stimulation signal is less than the second preset value, increasing a magnitude of current in the pulsed TMS device, until the difference between the intensity of the stimulation signal and the second preset value is less than the preset difference.

8. The method of claim 6, wherein adjusting the magnitude of current in the pulsed TES device and the pulsed TMS device based on the comparison result comprises:

adjusting a magnitude of current in the pulsed TES device and the pulsed TMS device respectively based on a preset ratio in the case that the comparison result is that the difference between the intensity of the stimulation signal and the second preset value is greater than the preset difference.

9. An electromagnetic stimulation device, comprising:

a receiving module, used for receiving an operation instruction input from a user; and

a control module, used for in response to the operation instruction, performing a control, in the case that the operation instruction is used to indicate deep stimulation of a target area of a target object, that a current direction of a pulsed transcranial electrical stimulation (TES) device of an electromagnetic stimulation apparatus inside the

EP 4 548 966 A1

target area is the same as a current direction of a pulsed transcranial magnetic stimulation (TMS) device of the electromagnetic stimulation apparatus inside the target area to electromagnetically stimulate the target area through current signals generated in the target area by the pulsed TES device and the pulsed TMS device, wherein a stimulation intensity corresponding to the deep stimulation is greater than a first preset value; wherein the control module is further used for performing a control, in the case that the operation instruction is used to indicate shallow stimulation of the target area, that a current direction of the pulsed TES device inside the target area is opposite to a current direction of the pulsed TMS device inside the target area to electromagnetically stimulate the target area through current signals generated in the target area by the pulsed TES device and the pulsed TMS device, wherein a stimulation intensity corresponding to the shallow stimulation is less than or equal to the first preset value.

10. An electromagnetic stimulation apparatus, comprising: a pulsed transcranial electrical stimulation (TES) device, a pulsed transcranial magnetic stimulation (TMS) device and a controller, wherein the controller is connected to the pulsed TES device and the pulsed TMS device respectively, and the controller is used for performing the electromagnetic stimulation method of any one of claims 1 to 8.

11. The electromagnetic stimulation apparatus of claim 10, wherein the pulsed TES device comprises a TES controller, a digital-to-analog conversion module, a current source, and multiple electrode pairs, each electrode pair comprising a positive electrode and a negative electrode;

the digital-to-analog conversion module is connected to the TES controller and a first end of the current source respectively; the digital-to-analog conversion module is used for performing digital-to-analog conversion on a control signal transmitted from the TES controller, and transmitting a converted analog signal to the current source; and a second end of the current source is connected to the positive electrode in each electrode pair, and a third end of the current source is connected to the negative electrode in each electrode pair, and the current source is used for outputting a current to the electrode pair based on the analog signals.

12. The electromagnetic stimulation apparatus of claim 11, wherein the second end of the current source is connected to the positive electrode in each electrode pair through a first resistor, and the third end of the current source is connected to the negative electrode in each electrode pair through a second resistor, wherein a first resistor connected to the positive electrode in an electrode pair has a same resistance value as a second resistor connected to the negative electrode in the same electrode pair, and all of resistance values of first resistors or second resistors corresponding to different electrode pairs are not the same.

13. The electromagnetic stimulation apparatus of claim 10, wherein the pulsed TES device comprises a TES controller, multiple power isolation modules, multiple digital-to-analog conversion modules, multiple current sources and multiple electrode pairs, each electrode pair comprises a positive electrode and a negative electrode, and the power isolation modules, the digital-to-analog conversion modules and the current sources are in one-to-one correspondence with the electrode pairs;

the TES controller is connected to a first end of each power isolation module respectively, a second end of each power isolation module is connected to a first end of a corresponding digital-to-analog conversion module, and a second end of the digital-to-analog conversion module is connected to a first end of a corresponding current source; a second end of each current source is connected to a positive electrode in a corresponding electrode pair, and a third end of the current source is connected to a negative electrode in the corresponding electrode pair; the digital-to-analog conversion module is used for performing digital-to-analog conversion on a control signal transmitted from the TES controller, and transmitting a converted analog signal to a corresponding current source; and the current source is used for outputting a current to the corresponding electrode pair based on the analog signals.

14. The electromagnetic stimulation apparatus of claim 10, wherein the pulsed TES device comprises a TES controller, multiple digital-to-analog conversion modules, multiple current sources, a ground electrode and multiple electrodes, and the digital-to-analog conversion modules and the current sources are in one-to-one correspondence with the electrodes;

the TES controller is connected to a first end of each digital-to-analog conversion module, a second end of each digital-to-analog conversion module is connected to a first end of a corresponding current source, a second end of each current source is connected to a corresponding electrode, and third ends of all current sources are connected to the ground electrode;

the digital-to-analog conversion module is used for performing digital-to-analog conversion on a control signal transmitted from the TES controller, and transmitting a converted analog signal to the corresponding current source; and

the current source is used for outputting a current to the corresponding electrode based on the analog signals.

15. A non-transitory computer-readable storage medium storing a computer program, wherein the computer program, when executed by a processor, performs the electromagnetic stimulation method of any one of claims 1 to 8.

FIG. 1

FIG. 2

FIG. 3

FIG. 4

FIG. 5a

FIG. 5b

FIG. 6a

FIG. 6b

FIG. 7a

FIG. 7b

| | |
|---|---|
| Receiving an operation instruction input from a user | 801 |

In response to the operation instruction, performing a control, in the case that the operation instruction is used to indicate deep stimulation of a target area of a target object, that a current direction of a pulsed TES device of the electromagnetic stimulation apparatus inside the target area is the same as a current direction of a pulsed TMS device of the electromagnetic stimulation apparatus inside the target area to electromagnetically stimulate the target area through current signals generated in the target area by the pulsed TES device and the pulsed TMS device — 802

Performing a control, in the case that the operation instruction is used to indicate shallow stimulation of the target area, that a current direction of the pulsed TES device inside the target area is opposite to the current direction of the pulsed TMS device inside the target area to electromagnetically stimulate the target area through current signals generated in the target area by the pulsed TES device and the pulsed TMS device — 803

FIG. 8

FIG. 9

FIG. 10

FIG. 11

FIG. 12

FIG. 13

FIG. 14

FIG. 15

FIG. 16

FIG. 17

FIG. 18

FIG. 19

FIG. 20

FIG. 21

FIG. 22

FIG. 23

FIG. 24

FIG. 25a

FIG. 25b

FIG. 25c

FIG. 26

FIG. 27

| | | | |
|---|---|---|---|
| TES controller | Digital-to-analog conversion DAC | Current source 1 | Electrode 1 |
| | Digital-to-analog conversion DAC | Current source 2 | Electrode 2 |
| | Digital-to-analog conversion DAC | Current source n | Electrode n |
| | | | Ground electrode |

FIG. 28

```
┌──────────────────────────────────────────────┐
│        ⌐11                ⌐12                  │
│   ┌───────────┐      ┌───────────┐             │
│   │ Receiving │      │Controlling│             │
│   │  module   │──────│  module   │             │
│   └───────────┘      └───────────┘             │
│        Electromagnetic stimulation             │
│                  device                        │
└──────────────────────────────────────────────┘
```

FIG. 29

## INTERNATIONAL SEARCH REPORT

| International application No. |
|---|
| **PCT/CN2022/125020** |

### A. CLASSIFICATION OF SUBJECT MATTER

A61N2/04(2006.01)i

According to International Patent Classification (IPC) or to both national classification and IPC

### B. FIELDS SEARCHED

Minimum documentation searched (classification system followed by classification symbols)

A61N

Documentation searched other than minimum documentation to the extent that such documents are included in the fields searched

Electronic data base consulted during the international search (name of data base and, where practicable, search terms used)

CNTXT, ENTXT, CNKI, IEEE: 磁刺激, 刺激, 强度, 电刺激, 电流, 方向, 流向, 电极, electrical, stimulation, magnetic, intensity, current, direction, tes, tms, electrode

### C. DOCUMENTS CONSIDERED TO BE RELEVANT

| Category* | Citation of document, with indication, where appropriate, of the relevant passages | Relevant to claim No. |
|---|---|---|
| PX | CN 114796875 A (INSTITUTE OF AUTOMATION, CHINESE ACADEMY OF SCIENCES) 29 July 2022 (2022-07-29)<br>claims 1-15 | 1-15 |
| X | CN 107929938 A (XUANWU HOSPITAL, CAPITAL MEDICAL UNIVERSITY) 20 April 2018 (2018-04-20)<br>description, paragraphs [0043]-[0095], and figures 1-4 | 1-5, 9, 10, 15 |
| X | CN 111686374 A (TIANJIN TAIMUSI MEDICAL TECHNOLOGY CO., LTD.) 22 September 2020 (2020-09-22)<br>description, paragraphs [0021]-[0033], and figure 1 | 1-5, 9, 10, 15 |
| A | CN 114042251 A (NATIONAL RESEARCH CENTER FOR REHABILITATION TECHNICAL AIDS) 15 February 2022 (2022-02-15)<br>entire document | 1-15 |
| A | US 2014235927 A1 (BRAINSWAY, INC.) 21 August 2014 (2014-08-21)<br>entire document | 1-15 |

☐ Further documents are listed in the continuation of Box C.   ☑ See patent family annex.

| * | Special categories of cited documents: | | |
|---|---|---|---|
| "A" | document defining the general state of the art which is not considered to be of particular relevance | "T" | later document published after the international filing date or priority date and not in conflict with the application but cited to understand the principle or theory underlying the invention |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **16 March 2023** | **22 March 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| Facsimile No. **(86-10)62019451** | Telephone No. |

Form PCT/ISA/210 (second sheet) (July 2022)

INTERNATIONAL SEARCH REPORT
Information on patent family members

International application No.

**PCT/CN2022/125020**

| Patent document cited in search report | | | Publication date (day/month/year) | Patent family member(s) | | | Publication date (day/month/year) |
|---|---|---|---|---|---|---|---|
| CN | 114796875 | A | 29 July 2022 | None | | | |
| CN | 107929938 | A | 20 April 2018 | None | | | |
| CN | 111686374 | A | 22 September 2020 | None | | | |
| CN | 114042251 | A | 15 February 2022 | None | | | |
| US | 2014235927 | A1 | 21 August 2014 | ES | 2899161 | T3 | 10 March 2022 |
| | | | | CA | 2901959 | A1 | 28 August 2014 |
| | | | | EP | 2958622 | A1 | 30 December 2015 |
| | | | | WO | 2014128632 | A1 | 28 August 2014 |
| | | | | IL | 240726 | A0 | 29 October 2015 |
| | | | | AU | 2014220310 | A1 | 15 October 2015 |
| | | | | JP | 2016507324 | A | 10 March 2016 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202210754796